# EUROPEAN PATENT SPECIFICATION

(11) **EP 2 579 205 B1**
(45) Date of publication and mention of the grant of the patent: **13.07.2016**
(21) Application number: 11792211.2
(22) Date of filing: 31.03.2011
(51) Int. Cl.: G06Q 50/00, C12M 3/00, C12N 5/00, G06F 19/00

(54) **CELL SORTER, CELL-SORTING METHOD AND PROGRAM**
ZELLSORTIERER, ZELLSORTIERUNGSVERFAHREN UND PROGRAMM DAFÜR
TRIEUR DE CELLULES, PROCÉDÉ ET PROGRAMME DE TRI DE CELLULES

(30) Priority: 07.06.2010 JP 2010130513
(43) Date of publication of application: 10.04.2013
(73) Proprietor: Okinawa Institute of Science and Technology Graduate University, Okinawa 904-0495 (JP); The Systems Biology Institute, Tokyo 108-0071 (JP)
(72) Inventor: KITANO, Hiroaki, Kunigami-gun, Okinawa 9040495 (JP)
(74) Representative: Hoffmann Eitle
(86) International application number: PCT/JP2011/058299
(87) International publication number: WO 2011/155253

(56) References cited:
- JP-A- 2004 520 829
- JP-A- 2007 517 524
- US-A1- 2007 298 411
- HIROAKI KITANO: "Violations of robustness trade-offs", MOLECULAR SYSTEMS BIOLOGY, vol. 6, 22 June 2010 (2010-06-22), XP055107630, DOI: 10.1038/msb.2010.40
- EDMUND, D. ET AL.: 'Stochastic Combinatorial Optimization Approach to Biopharmaceutical Portfolio Management' INDUSTRIAL & ENGINEERING CHEMISTRY RESEARCH vol. 47, no. 22, 28 October 2008, pages 8762 - 8774, XP055107613
- H KITANO: 'Towards a theory of biological robustness' MOLECULAR SYSTEMS BIOLOGY vol. 3, no. 137, 18 September 2007, pages 1 - 7, XP055107626
- H KITANO: 'Violations of robustness trade-offs, art 384' MOLECULAR SYSTEMS BIOLOGY vol. 6, 22 June 2010, pages 1 - 8, XP055107630

## Description

### TECHNICAL FIELD

The present invention relates to a cell selecting apparatus, a cell selecting method, and a computer program product.

### BACKGROUND ART

A technique for analysis of cell culture data and the like is described by conventional methods relating to bioinformatics.

For example, according to the analytical methods disclosed in Non Patent Literature 1 and Non Patent Literature 2, an analytical technique using GI50 (50% Growth Inhibition), which indicates the sensitivity of an anti-cancer agent against cancer cells cultured under a certain culture condition, and cluster analysis is disclosed.

Further, a method for diversified investment of assets is disclosed by conventional investment theories.

For example, according to the portfolio selection adopted for modern investment theories described in Non Patent Literature 3, a method of lowering investment risk by combining plural investment options is disclosed. Specifically, since financial goods with high expected return (profit) are generally accompanied with high risk while financial goods with low profit have low risk, it is believed in the modern investment theories that a tradeoff is present between the expected return and the risk (deviation in return represented by standard deviation). Thus, with regard to the portfolio selection, a method of finding an optimum combination (portfolio) having desirable profit rate and acceptable risk among the financial goods (investment goods) each having different expected profit rate and risk is suggested for an investor or others who purchase multiple financial goods.
Non Patent Literature 1: Nakatsu N, Yoshida Y, Yamazaki K, Nakamura T, Dan S, Fukui Y, Yamori T. Chemosensitivity profile of cancer cell lines and identification of genes determining chemosensitivity by an integrated bioinformatical approach using cDNA arrays. Mol Cancer Ther. 2005 Mar; 4(3): 399-412.
Non Patent Literature 2: Dan S, Tsunoda T, Kitahara O, Yanagawa R, Zembutsu H, Katagiri T, Yamazaki K, Nakamura Y, Yamori T. An integrated database of chemosensitivity to 55 anticancer drugs and gene expression profiles of 39 human cancer cell lines. Cancer Res. 2002 Feb 15; 62(4): 1139-47.
Non Patent Literature 3: Markowitz, H.M. (1991) Portfolio selection: efficient diversification of investments. John Wiley & Sons.

Background art is also disclosed in US 2007/0298411 A1, specifically a method for determining the effect of a plurality of culture conditions on a cell, which comprising the steps of: a) providing a first set of groups of cell units each comprising one or more cells, and exposing said groups to desired culture conditions; (b) pooling two or more of said groups to form at least one second pool; (c) subdividing the second pool to create a further set of groups of cell units; (d) exposing said further groups to desired culture conditions; (e) optionally, repeating steps (b)-(d) iteratively as required; and (f) optionally assessing the effect on a given cell unit of the culture conditions to which it has been exposed.

### SUMMARY

However, according to the analytical method described in Non Patent Literature 1 and Non Patent Literature 2, presence or absence of proliferation inhibition was examined by administering an anti-cancer agent to each cancer cells cultured under fixed culture condition that is different from the diverse environments inside a living body. For such reasons, according to the analytical method, only the cells fitted to the fixed culture condition (over adaptation) can proliferate and survive selectively, while other cells are reduced in accordance with the passage. Thus, according to the analytical method, there is a problem that during the screening of an anti-cancer agent it is difficult to specify an anti-cancer agent or a combination of an anti-cancer agent, which can stably inhibit cancers under various environments.

Further, as the conventional portfolio selection described in Non Patent Literature 3 is directed only to a method of reasonable and diversified investment of financial goods, an application to bioinformation is not considered at all. Thus, there is a problem that, even when bioinformation is simply linked to portfolio selection, it cannot determine an optimum combination of cells or the like.

The present invention is devised in view of the problems described above, and an object of the invention is to provide a cell selecting apparatus, a cell selecting method, and a computer program product for selecting an optimum combination of cells or microorganisms by mapping, in a yield risk space, a portfolio including production outputs based on cell proliferation rate, production of biomass by microorganisms, or the like, and variables indicating the extent of the variation of production outputs, that are calculated from suppressed proliferation, reduced production outputs, or the like considering multiple disturbances and probability of its occurrence.

The invention in its general sense is an apparatus as set out in Claim 1, a method as set out in Claim 11, and a computer-readable medium as set out in Claim 12. Optional features are set out in the remaining claims.

### BRIEF DESCRIPTION OF DRAWINGS

FIG. 1 is a flow chart for illustrating the basic principle of the embodiment of the invention.
FIG. 2 is a diagram for illustrating an example of a yield risk space according to the embodiment of the invention.
FIG. 3 is a diagram for illustrating an example of probability distribution of expected yield against the production outputs and portfolio of each cells according to the embodiment of the invention.
FIG. 4 is a diagram for illustrating an example of indifference curve according to the embodiment of the invention.
FIG. 5 is a diagram for illustrating an example of indifference curve according to the embodiment of the invention.
FIG. 6 is a block diagram for illustrating an example of constitution of the cell selecting apparatus to which the embodiment of the invention is applied.
FIG. 7 is a flow chart for illustrating an example of process of the cell selecting apparatus according to the embodiment of the invention.
FIG. 8 is a flow chart for illustrating an example of process of the cell selecting apparatus according to the embodiment of the invention.
FIG. 9 is a diagram for illustrating an example of change in increase ratio of the cells that is caused by different disturbance in the embodiment of the invention.
FIG. 10 is a diagram for illustrating an example of change in increase ratio of the cells that is caused by different disturbance in the embodiment of the invention.
FIG. 11 is a diagram for illustrating an example of change in increase ratio of the cells that is caused by different disturbance in the embodiment of the invention.
FIG. 12 is a diagram for illustrating an example of change in cell state that is caused by different disturbance in the embodiment of the invention.
FIG. 13 is a diagram for illustrating an example of change in cell state that is caused by different disturbance in the embodiment of the invention.
FIG. 14 is a diagram for illustrating an example of the measured data according to the embodiment of the invention.
FIG. 15 is a concept diagram for illustrating an example of multi-point sampling of cells according to the embodiment of the invention.
FIG. 16 is a diagram for illustrating an example of projection into the yield risk space according to the embodiment of the invention.

### DESCRIPTION OF EMBODIMENTS

Herein below, embodiments for carrying out the cell selecting apparatus, cell selecting method, and computer program product according to the invention will be described in detail with reference to the drawings. However, the invention is not limited to the embodiments but is defined in the claims.

In the embodiments given below, an example of applying the invention to pharmaceuticals and biomass production will be described, in particular. However, the present invention is not limited thereto, the same application can be made to all technical fields in which cell culture is employed.

There is described in the following a cell selecting apparatus comprising at least a storage unit and a control unit, wherein the storage unit includes a measured data storage unit that stores, for each cells, measured data about a proliferation of the cells cultured under multiple conditions or a production of biomass, and the control unit includes a setting unit that sets, for each cells or each condition, a production output of the cells or the biomass based on the measured data that are stored in the measured data storage unit and a variable indicating an extent of variation of the production output, a variable range calculating unit that calculates a variable range of the production output and the variable in case of combining the multiple cells or conditions based on the production output and the variable set by the setting unit, and an optimizing unit that calculates an optimum combination within the variable range by using an optimization method.

In the cell selecting apparatus the variable may be a standard deviation of the production output.

Furthermore, the variable range calculating unit may calculate, based on the production output and the variable, a variable range of the production output and the variable in case of combining the multiple cells or conditions on the basis of covariance.

Furthermore, the optimizing unit may calculate a combination closest to effective frontier for maximizing the production output under the constant variable within the variable range by using the optimization method.

The optimization method may be Pareto optimization, genetic algorithm, least square method, or a suitable probabilistic or analytical optimization method.

In the cell selecting apparatus described herein, the multiple cells may be cells of multiple types. Alternatively, the multiple cells may be multiple cells of a single type. In either case, the cells may be microorganisms or cancer cells.

In the cell selecting apparatus described herein, the measured data may be GI50 that is a concentration of a compound for inhibiting cell proliferation by 50% and the production output may be a reciprocal number of GI50.

There is also described in the following a cell selecting method executed by a cell selecting apparatus including at least a storage unit and a control unit, wherein the storage unit includes a measured data storage unit that stores, for each cells, measured data about a proliferation of the cells cultured under multiple conditions or a production of biomass, the method executed by the control unit comprising a setting step of setting, for each cells or each condition, a production output of the cells or the biomass based on the measured data that are stored in the measured data storage unit and a variable indicating an extent of variation of the production output, a variable range calculating step of calculating a variable range of the production output and the variable in case of combining the multiple cells or conditions based on the production output and the variable set at the setting step, and an optimizing step of calculating an optimum combination within the variable range by using an optimization method.

There is also described a computer program product having a non-transitory computer readable mediums including programmed instructions for a cell selecting method executed by a cell selecting apparatus including at least a storage unit and a control unit, wherein the storage unit includes a measured data storage unit that stores, for each cells, measured data about a proliferation of the cells cultured under multiple conditions or a production of biomass, wherein the instructions, when executed by the control unit, cause the control unit to execute a setting step of setting, for each cells or each condition, a production output of the cells or the biomass based on the measured data that are stored in the measured data storage unit and a variable indicating an extent of variation of the production output, a variable range calculating step of calculating a variable range of the production output and the variable in case of combining the multiple cells or conditions based on the production output and the variable set at the setting step, and an optimizing step of calculating an optimum combination within the variable range by using an optimization method.

Because the technique described herein includes setting, for each cells or each condition, a production output of the cells or the biomass based on the measured data and a variable indicating an extent of variation of the production output, calculating a variable range of the production output and the variable in case of combining the multiple cells or conditions based on the production output and the variable, and calculating an optimum combination within the variable range by using an optimization method, for producing antibiotics and biomass such as bioethanol, an optimum combination of microorganisms can be found for a plant in which the conditions are easily varies, and therefore an effect of achieving effective operation of biomass production can be obtained. Further, for screening of pharmaceuticals such as anti-cancer agent, an effect of developing a new pharmaceutical for efficient cancer inhibition can be obtained regardless of many differences among individuals, inside body environments, and the like.

Further, where the variable is standard deviation of the production output, an effect of enabling the numeric expression of a deviation of cell proliferation rate or biomass production outputs can be obtained.

Further, where the technique involves calculating, based on the production output and the variable, a variable range of the production output and the variable in case of combining the multiple cells or conditions on the basis of covariance, an effect of enabling the setting of a portfolio selectable region considering correlation and anti-correlation between cell lines can be obtained.

Further, where a combination closest to effective frontier for maximizing the production output under the constant variable within the variable range is calculated by using the optimization method, an effect of enabling the selection of a combination of cells and the like that is close to the combination of cells and the like performing the theoretically most effective production under constant risk can be obtained.

Further, where the optimization method includes Pareto optimization, genetic algorithm, least square method, or a suitable probabilistic and analytical optimization method, an effect of enabling the shortening of process time can be obtained by using a general calculation method.

Further, where the multiple cells are the cells of multiple types, an effect of enabling the application on screening of an anti-cancer agent and the like which can efficiently suppress proliferation of a group of cancer cells and the like having genetic diversity can be obtained.

Further, where the multiple cells are the multiple cells of single type, an effect of enabling the specification of a combination of conditions and the like for efficient proliferation of specific cells can be obtained.

Further, where the cells are microorganisms, an effect of enabling the utilization for production of antibiotics, bioethanol, and the like can be obtained.

Further, where the cells are cancer cells, an effect of enabling the utilization for development of an anti-cancer agent can be obtained.

Further, the measured data are GI50, i.e., concentration of a compound for inhibiting cell proliferation by 50%, and the production outputs are a reciprocal number of GI50, an effect of enabling the specification of an optimum combination of cells or anti-cancer agent by using the data accumulated all over the world until now can be obtained by using generic indices even when the measured data itself is small.

### Outline of Embodiment of Invention

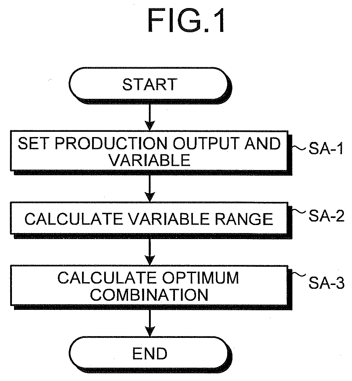

Outline of the embodiment of the invention will be described with reference to FIG. 1 to FIG. 5. After that, constitution, process, and the like according to the embodiment are described in greater detail. FIG. 1 is a flow chart for illustrating the basic principle of the embodiment.

Briefly, the present embodiment has basic characteristics as follows. That is, as illustrated in FIG 1, for each cells or condition, a control unit of the cell selecting apparatus according to the present embodiment sets production outputs (yield) of cells or biomass based on measured data of proliferation of cells, which have been cultured under the multiple conditions or production of biomass stored in the storage unit, and variables (risk) indicating the extent of the variation of the production outputs (Step SA-1). As described herein, the variables may be standard deviation of the production outputs. Further, as for the control unit, the production outputs may be set under the consideration of disturbance and probability of cell generation for each culture condition.

In addition, the control unit of the cell selecting apparatus calculates, based on the production outputs (yield) and variables (risk) set in the step SA-1, variable ranges (portfolio selectable ranges) of the production outputs (yield) and variables (risk) in case of forming a combination (portfolio) of multiple cells or conditions (step SA-2). In this regard, the control unit may calculate, based on the production outputs and variables, the variable ranges of the production outputs and variables in case of combining multiple cells or conditions on the basis of covariance. Further, the control unit may calculate, based on the production outputs and variables, the variable ranges of the production outputs and variables in case of combining multiple cells or conditions by calculating the correlation and anti-correlation between cells using the covariance calculation method. Further, the multiples cells may be cells of multiple types or multiple cells of a single type.

In addition, the control unit of the cell selecting apparatus calculates an optimum combination of cells or conditions (portfolio selection) within the variable ranges (portfolio selectable ranges) by using an optimization method (step SA-3) and ends the process. Herein, the control unit may calculate a combination closest to effective frontier for maximizing the production outputs under constant variables within the variable ranges by using an optimization method. Further, the optimization method can be Pareto optimization, genetic algorithm, least square method, or other suitable probabilistic or analytical optimization method.

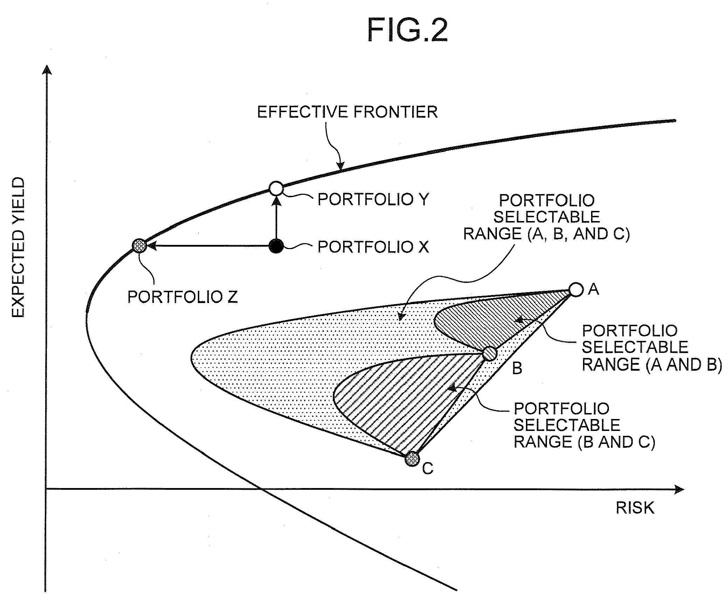

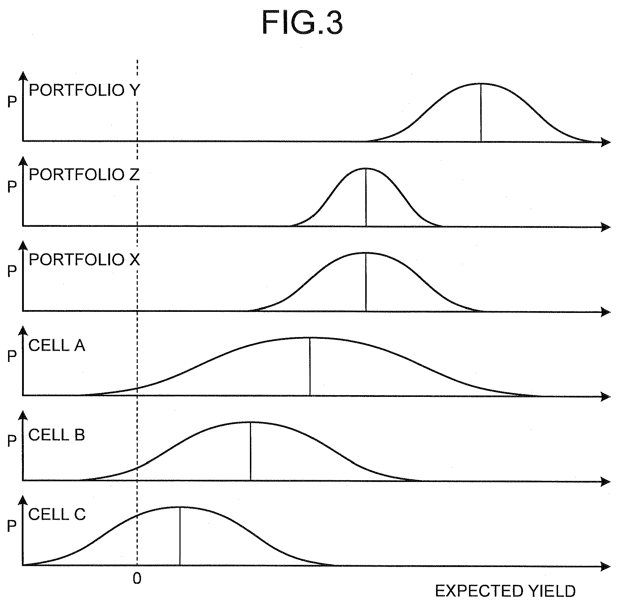

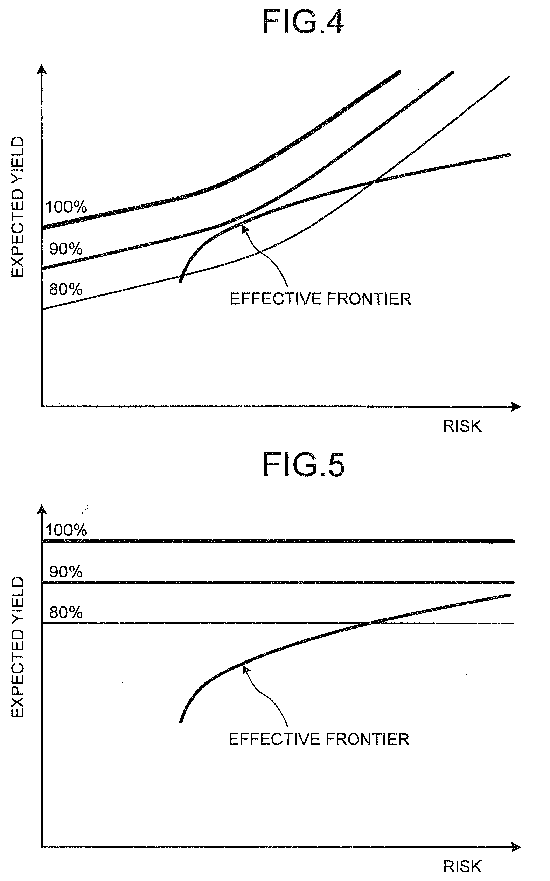

Herein below, basic concept for portfolio selection will be described with reference to FIG. 2 to FIG. 5. FIG. 2 is a diagram for illustrating an example of a yield risk space according to the embodiment. FIG. 3 is a diagram for illustrating an example of probability distribution of expected yield against the production outputs and portfolio of each cells according to the embodiment. FIG. 4 is a diagram for illustrating an example of indifference curve according to the embodiment. FIG. 5 is a diagram for illustrating an example of indifference curve according to the embodiment.

As illustrated in FIG. 2, by mapping any combination of cells (portfolio) in a yield risk space, the portfolio selection can be visualized. Herein, the yield (performance) is represented by the production outputs of cells and the risk is represented by the variation in the production outputs.

Herein, as illustrated in FIG. 3, the expected yield is obtained as expected value from statistical variance of the yield for each cells A, B, and C, and the yield for cell combination portfolio (portfolio, X, Y, and Z).

Back to FIG. 2, the yield and risk obtained for cells A, B, and C can be expressed as points A, B, and C in the yield risk space. Further, in FIG. 2, each combination (portfolio) of points A, B, and C can be obtained as a variable range (portfolio selectable range) which has, in an yield risk space, effective frontier on the outer periphery.

In FIG. 2, the portfolio X is inferior to the portfolio Y and portfolio Z on the effective frontier, for example. In other words, in the same risk, the portfolio Y has higher expected yield than the portfolio X (i.e., the portfolio X has lower expected yield in the same risk). Further, in the same expected yield, the portfolio Z has lower risk than the portfolio X (i.e., the portfolio X has higher risk in the same expected yield).

As used herein, the effective frontier indicates a group of points in a yield risk space which consists of an optimum combination of cells for maximizing the expected yield under constant risk. A combination of cells outside the effective frontier is inferior to the combination present on the effective frontier. However, on the effective frontier, since the change in expected yield is directly linked to a change in risk, a tradeoff between risk and expected yield occurs in the optimized portfolio present on the effective frontier. Further, in theory, any portfolio not present on the effective frontier can maximize the expected yield without increasing the risk or can reduce the risk without lowering the expected yield. For example, as shown by the arrow extended from the portfolio X illustrated in FIG. 2, the portfolio X can be re-constructed to be close to the effective frontier.

Further, as illustrated in FIG. 4 and FIG. 5, by defining an indifference curve, which is a group of points with the same effect specifically under risk preference condition, in a yield risk space, evaluation of portfolio value (optimization) can be performed. Accordingly, selection of portfolio which is on the effective frontier and also on the indifference curve can be made. In other words, by using the indifference curve of risk avoiding type illustrated in FIG. 4, portfolio selection for maximizing the expected yield while avoiding risk can be made. Still further, by using a risk neutral type indifference curve illustrated in FIG. 5, portfolio selection for a case in which only maximization of expected yield is concerned can be made.

As above, descriptions for the outline of the embodiment have been explained.

### Constitution of Cell Selecting Apparatus 100

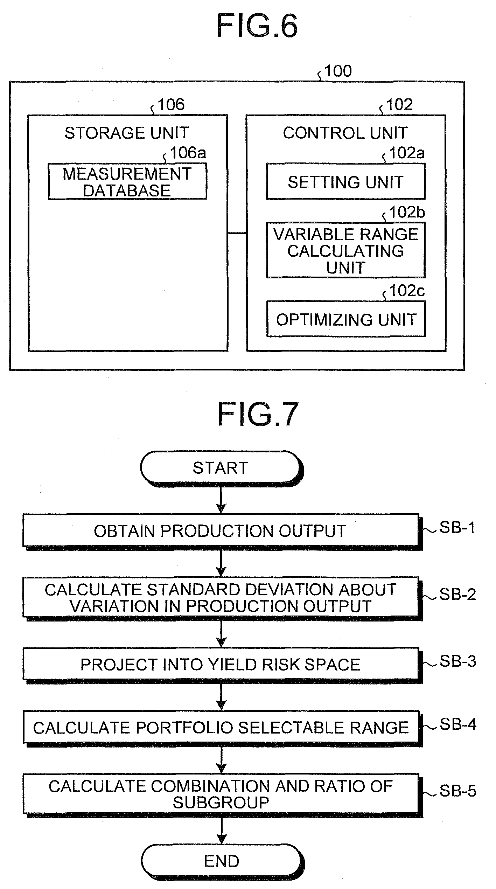

Next, constitution of a cell selecting apparatus 100 will be described with reference to FIG. 6. FIG. 6 is a block diagram for illustrating an example of constitution of the cell selecting apparatus 100 to which the embodiment is applied, and for the constitution, only the parts relating to the embodiment are conceptually illustrated.

In FIG. 6, the cell selecting apparatus 100 includes a control unit 102 and a storage unit 106 in brief. Herein, the control unit 102 is a CPU or the like which controls the entire cell selecting apparatus 100 on the whole. Further, the storage unit 106 is a device for storing various database, tables, and the like. Each unit of the cell selecting apparatus 100 is connected so that it can communicate with each other via any communication pathway. Further, the cell selecting apparatus 100 can be connected such that it can communicate with a network via a communication device such as a router and a wired or wireless communication line such as dedicated line.

Various database or tables that are stored in the storage unit 106 (a measurement database 106a) are a storage unit such as a fixed disc device. For example, the storage unit 106 stores various programs, tables, files, database, and web pages or the like that are used for various processes.

Among the constitutional elements of the storage unit 106, the measurement database 106a is a measured data storage unit, that stores, for each cells, measured data regarding proliferation of cells cultured under multiple conditions (culture condition, disturbance, and the like) or production of biomass (for example, indices or the like to be adopted as yield (production outputs) such as proliferation rate by each cells under each condition, in which each cells has been cultured under multiple conditions, or biomass production). Herein, the cell can be a microorganism. Further, the cell can be a cancer cell. Further, the measured data can be GI50, which indicates the concentration of a compound inhibiting the cell proliferation by 50%.

Further, in FIG. 6, the control unit 102 has a control program such as Operating System (OS), a program for defining order of various processes and the like, and an internal memory for storing data for use. Based on those programs and the like, the control unit 102 performs the information process for performing various processes. In terms of functional concept, the control unit 102 has a setting unit 102a, a variable range calculating unit 102b, and an optimizing unit 102c.

Among those described above, the setting unit 102a is a setting unit that sets production outputs of cells or biomass based on the measured data that are stored in the measurement database 106a and variables for each cells or condition in which the variables indicate the extent of the variation of the production outputs. Herein, the variables can be standard deviation of the production outputs. Further, the production outputs can be reciprocal number of GI50. Further, the setting unit 102a may set the production outputs by considering the disturbance may probability of cell generation for each culture condition.

Further, the variable range calculating unit 102b is a variable range calculating unit that calculates the variable ranges of production outputs and variables in case of combining multiple cells or conditions, based on the production outputs and variables that are set by the setting unit 102a. Herein, based on the production outputs and variables, the variable range calculating unit 102b may calculate the variable ranges of production outputs and variables in case of combining multiple cells or conditions on the basis of covariance. Further, based on the production outputs and variables, the variable range calculating unit 102b may calculate the correlation and anti-correlation among cells by a method for calculating covariance and calculate the variable ranges of production outputs and variables in case of combining multiple cells or conditions. Further, the multiple cells may be cells of multiple types or multiple cells of a single type.

Still further, the optimizing unit 102c is an optimizing unit that calculates, by using an optimization method, an optimum combination within the variable ranges. Herein, the optimizing unit 102c may calculate, using the optimization method, a combination closest to effective frontier for maximizing the production output under constant variables within the variable ranges. Further, the optimization method may be Pareto optimization, genetic algorithm, least square method, or any probabilistic or analytical optimization.

### Process of Cell Selecting Apparatus 100

Next, an example of the process of the system of the embodiment that is constituted as described above will be described in detail with reference to FIG. 7 to FIG. 16 given below.

### Process 1 (Production of Biomass)

First, details about the process with the cell selecting apparatus 100 in biomass production according to the embodiment will be described with reference to FIG. 7. FIG. 7 is a flow chart for illustrating an example of process of the cell selecting apparatus 100.

As illustrated in FIG. 7, the setting unit 102a obtains the production outputs of biomass (ethanol, antibiotics, or the like) for each microorganism (subgroups) cultured under multiple conditions (culture condition, disturbance, or the like) that are included in the measured data stored in the measurement database 106a from the measurement database 106a and sets the (mean value of) production outputs as yield for each subgroup (step SB-1). In this case, the measured data that are stored in the measurement database 106a may be production outputs of biomass obtained by culturing the microorganism (subgroup) having multiple genetic backgrounds under the (single or multiple) conditions. Further, the measured data that are stored in the measurement database 106a may be production outputs of biomass measured under multiple culture conditions corresponding to each condition, when the variation in presumed culture condition is identified and probability of each presumed occurrence is considered for the culture process.

Further, the setting unit 102a calculates the standard deviation about variation in production outputs of the biomass obtained in the step SB-1 and sets the calculated standard deviation as risk for each subgroup (step SB-2).

Further, the setting unit 102a projects (maps) the yield set in the step SB-1 and the risk set in the step SB-2 into a yield risk space by using the yield and the risk as a coordinate of subgroups (step SB-3).

Further, the variable range calculating unit 102b calculates correlation and anti-correlation among subgroups using a method for calculating covariance based on the coordinates for each subgroup projected into a yield risk space by the setting unit 102a in the step SB-3, and calculates the variable ranges (portfolio selectable ranges) of a combination (portfolio) of multiple subgroups (step SB-4).

Further, by performing optimization which uses a separately defined indifference curve in a yield risk space (for example, Pareto optimization, genetic algorithm, or least square method), the optimizing unit 102c calculates the ratio and the combination (portfolio) of a subgroup having the maximum effect as being closest to the effective frontier in the portfolio selectable ranges obtained by the calculation in the step SB-4 (step SB-5). Accordingly, an optimum operation condition for the biomass·plant can be set (for example, what kind of microorganisms should be cultured at which ratio in a tank in order to stably obtain the production outputs), and thus the invention is effective for optimum operation of a plant having variable culture conditions.

### Process 2 (Screening of Anti-Cancer Agent)

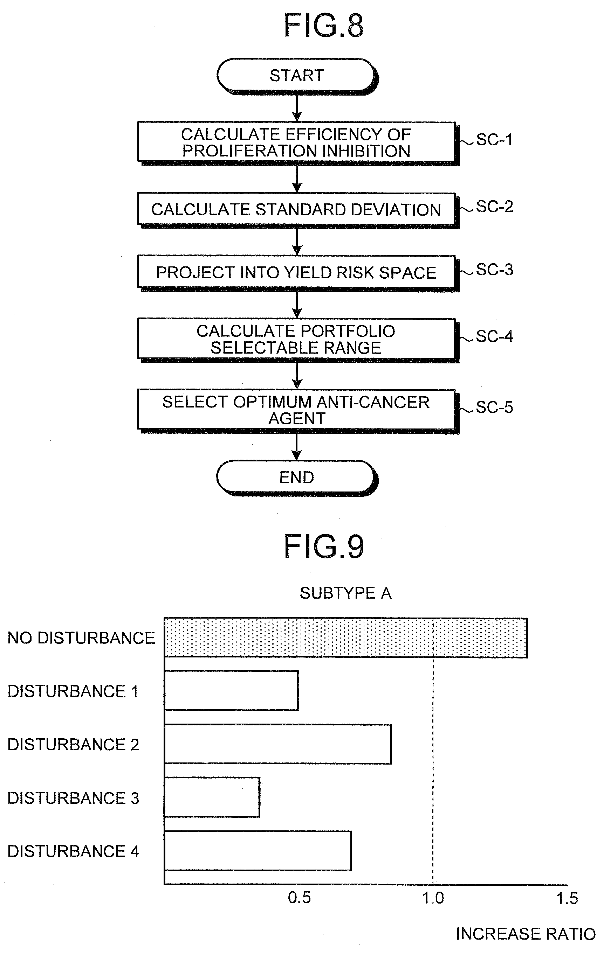

Next, details of the process of the cell selecting apparatus 100 for screening of an anti-cancer agent will be described with reference to FIG. 8 to FIG. 16. FIG. 8 is a flow chart for illustrating an example of process of the cell selecting apparatus 100 according to the embodiment.

As illustrated in FIG. 8, in screening of an anti-cancer agent, the setting unit 102a calculates (mean value of) reciprocal number of the GI50 for each cells as efficiency for proliferation inhibition of cancer cells based on measured data (GI50 is concentration of an anti-cancer agent which inhibits cancer cell proliferation by 50%) about proliferation of cancer cells which have been cultured under multiple conditions (for example, culture condition including administration of an anti-cancer agent, disturbance, and the like) stored in the measurement database 106a, and sets as production outputs (yield) for each cancer cells or anti-cancer agent (step SC-1). Herein, the yield (production outputs) may be inhibitory efficiency per milligram of an anti-cancer agent, i.e., efficiency for proliferation inhibition and the like of administered concentration with respect to maximum allowable concentration of an anti-cancer agent.

Herein, referring to FIG. 9 to FIG. 16, an example of the measured data that are stored in the measurement database 106a according to the embodiment will be described.

In general, for screening of an anti-cancer agent, cancer cells are cultured under a fixed culture condition that is different from the environment inside a living body. Since the cancer cells fitted to the fixed culture condition will exhibit the highest proliferation, even when the developments are made by having as a candidate for new pharmaceuticals a compound exhibiting an effect against the cancer cells suitable for the condition, there is a possibility that the results different from those expected may be obtained in real clinical practice. Thus, as measured data for the embodiment, it is possible that multiple disturbances in condition are presumed to prevent over adaptation to the culture condition and the measured data obtained from cancer cells that are actually cultured with certain probability are used. Accordingly, the measured data obtained from cancer cells according to the embodiment is not limited to the measured data obtained from over adaptation to culture condition, and as a result, screening of suitable anti-cancer agent can be made. Herein, examples of the disturbances in condition may include variation in factors which fluctuate depending on one-day period, such as NADP, ATP, and glucose, and periodic variation in gene expression. The disturbances in condition may be approximated by various methods such as change in medium composition, and introduction of RNAi to cells during the cell culture.

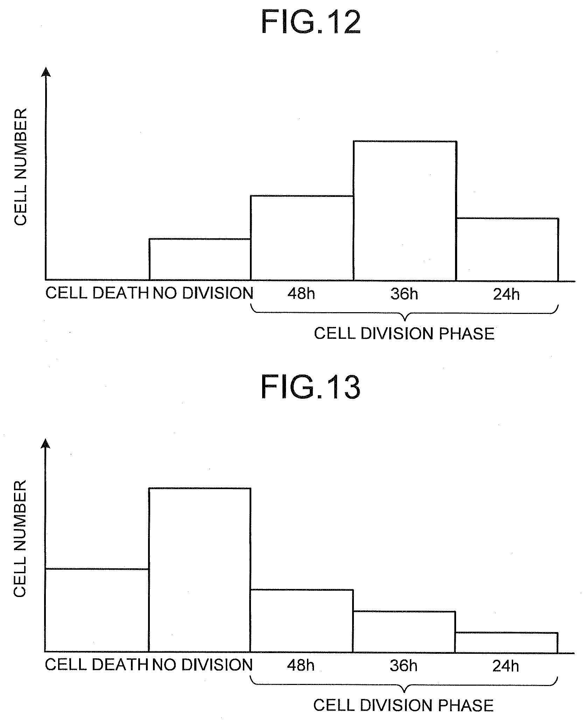

Herein, referring to FIG. 9 to FIG. 13, influences of the disturbances in condition on cell culture according to the embodiment of the invention will be described. FIG. 9 to FIG. 11 are diagrams for illustrating an example of change in increase ratio of the cells that is caused by different disturbance in the embodiment of the invention. FIG. 12 and 13 are diagrams for illustrating an example of change in cell state that is caused by different disturbance in the embodiment of the invention.

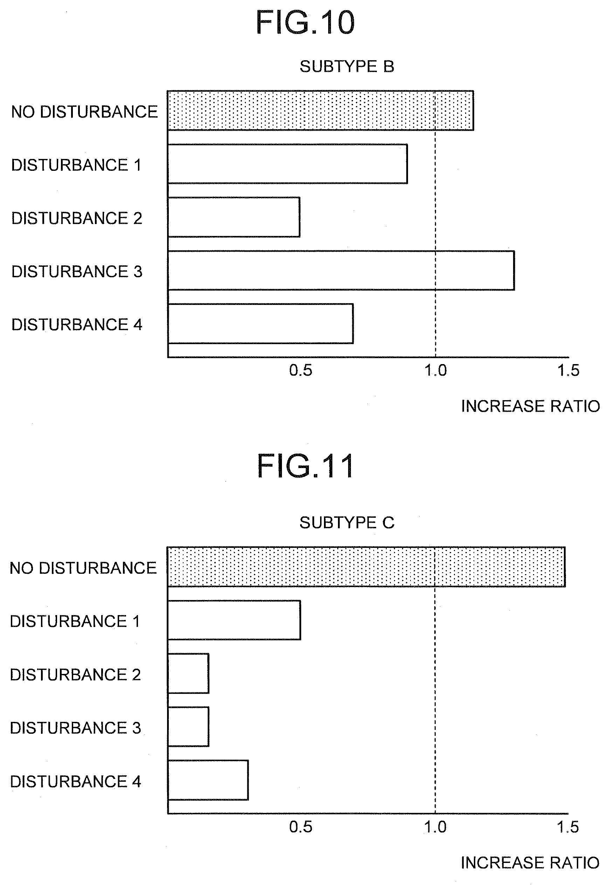

As illustrated in FIG. 9, cells of the subtype A have increase ratio of about 1.4 times compared to the average increase ratio of the entire cells when they are cultured in a state with no disturbance. They have increase ratio of about 0.5 times compared to the average increase ratio of the entire cells when cultured in a state with disturbance 1. They have increase ratio of about 0.8 times compared to the average increase ratio of the entire cells when cultured in a state with disturbance 2. They have increase ratio of about 0.3 times compared to the average increase ratio of the entire cells when cultured in a state with disturbance 3. They have increase ratio of about 0.7 times compared to the average increase ratio of the entire cells when cultured in a state with disturbance 4. Further, as illustrated in FIG. 10, cells of the subtype B have increase ratio of about 1.1 times compared to the average increase ratio of the entire cells when they are cultured in a state with no disturbance, for example. They have increase ratio of about 0.9 times compared to the average increase ratio of the entire cells when cultured in a state with disturbance 1. They have increase ratio of about 0.5 times compared to the average increase ratio of the entire cells when cultured in a state with disturbance 2. They have increase ratio of about 1.3 times compared to the average increase ratio of the entire cells when cultured in a state with disturbance 3. They have increase ratio of about 0.6 times compared to the average increase ratio of the entire cells when cultured in a state with disturbance 4. Further, as illustrated in FIG. 11, cells of the subtype C among the cells of the same type have increase ratio of about 1.5 times compared to the average increase ratio of the entire cells when they are cultured in a state with no disturbance, for example. They have increase ratio of about 0.5 times compared to the average increase ratio of the entire cells when cultured in a state with disturbance 1. They have increase ratio of about 0.2 times compared to the average increase ratio of the entire cells when cultured in a state with disturbance 2. They have increase ratio of about 0.2 times compared to the average increase ratio of the entire cells when cultured in a state with disturbance 3. They have increase ratio of about 0.3 times compared to the average increase ratio of the entire cells when cultured in a state with disturbance 4.

Herein, as a result of examining in more detail the cell group having increased ratio and the cell group having decreased ratio illustrated in FIG. 9, it was found that the cells of the subtype A which have been cultured in a state without disturbance have no cells undergone cell death and the number of cells in division phase is greater than the number of cells in non-division phase, as illustrated in FIG. 12. Meanwhile, as illustrated in FIG. 13, it was found that the cells of the subtype A which have been cultured in a state with disturbance 4 have no cells undergone cell death and the number of cells undergone cell death and cells in non-division phase is greater than the number of cells in division phase. Thus, as illustrated in FIG. 9 to FIG. 13, it was found that change in proliferation rate or the like is caused by disturbance among not only the cells of the same type but different subtypes but also the cells having the same genetic variation.

Accordingly, as the measured data stored in the measurement database 106a of the embodiment of the invention, data of proliferation rate measured by adding an anti-cancer agent for each condition of disturbance, and data obtained by measuring GI50 under each condition of disturbances by culturing each cancer cell line under multiple conditions of disturbance may be also used. For example, when several fragments under actual environment are cultured under C kinds of disturbance without an anti-cancer agent or with an anti-cancer agent (a candidate compound) of N types at D concentrations, culture can be carried out with C * (1 + N * D) kinds. Accordingly, having the culture results with no anti-cancer agent as a reference data, C * N * D kinds of GI50 of the cells can be calculated. Further, when it is believed that the presumed disturbance occurs at presumed probability at the time of administering an anti-cancer agent in the embodiment of the invention, an anti-cancer agent having small GI50 and small risk (standard deviation) will be the most desired one. Thus, by setting the combination of disturbances that are close to actual environment inside a living body and probability of the occurrence thereof, a compound believed to have the highest effect in actual environment can be predicted among the candidate anti-cancer agents.

In addition, the setting unit 102a, when culture condition of C types occurs with constant probability, the setting may be used for yield calculation. Further, the setting unit 102a may presume multiple cases with varying probability of occurrence and calculate yield for each case.

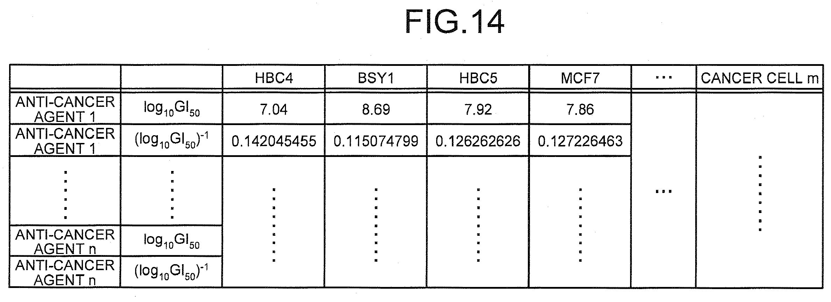

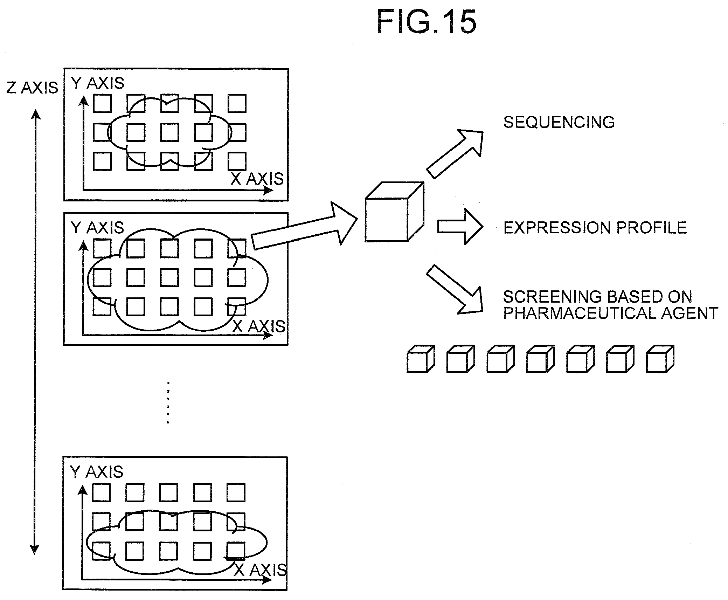

Referring to FIG. 14 and FIG. 15, one example of the measured data stored in the measurement database 106a of the embodiment of the invention will be described. FIG. 14 is a diagram for illustrating an example of the measured data according to the embodiment of the invention. FIG. 15 is a concept diagram for illustrating an example of multi-point sampling of cells according to the embodiment of the invention.

From various studies on cancer cells, it has been found that genetically diverse cancer cells are present as a mixture even in a tumor of single patient (Baisse B, Bouzourene H, Saraga EP, Bosman FT, Benhattar J. Intratumor genetic heterogeneity in advanced human colorectal adenocarcinoma. Int J Cancer. 2001 Aug 1; 93(3): 346-52.). For such reasons, there is a case in which one anti-cancer agent has an effect on cancer cells having specific mutation but has no effect on cancer cells having other mutation, that is, resistance to the anti-cancer agent may be exhibited. Thus, when an anti-cancer agent is administered in such state, cancer having an ability of maintaining proliferation even in the presence of the anti-cancer agent may be present at great ratio due to genetic diversity of cancer. Thus, as a way of coping with the genetic diversity of cancer, use of an additional anti-cancer agent, that is, use of a mixture of multiple anti-cancer agents, is required.

Thus, as illustrated in FIG. 14, as a measured data stored in the measurement database 106a of the embodiment of the invention, GI50 (for example, 7.04, 8.69, 7.92, and 7.86) obtained by administering the anti-cancer agent 1 to anti-cancer agent n (n: natural number) to m (m: natural number) types of cancer cells including HBC4, BSY1, HBC5, MCF7, and the like and converting the result into a log scale may be used. Further, as illustrated in FIG. 14, as a measured data stored in the measurement database 106a of the embodiment of the invention, the reciprocal number (for example, 0.142045455, 0.115074799, 0.126262626, 0.127226463, and the like) of GI50 obtained by administering the anti-cancer agent 1 to anti-cancer agent n (n: natural number) to m (m: natural number) types of cancer cells including HBC4, BSY1, HBC5, MCF7, and the like and converting the result into a log scale may be used. Although the type of the cancer cells is different in FIG. 14, for genetically diverse cancer cells present as a mixture in a single lump of tumor, it is also possible to use, as a measured data stored in the measurement database 106a of the embodiment of the invention, GI50 obtained by performing separate culture for each cell cluster using a certain marker and converting the result into a log scale for the cell cluster.

Further, the setting unit 102a may set the average value (0.1276) of reciprocal number of GI50 obtained by the conversion into a log scale as the yield (proliferation inhibition efficiency) of the anti-cancer agent 1 and the standard deviation (0.011) of GI50 obtained by the conversion into a log scale as the risk of the anti-cancer agent 1.

Further, as an example of the embodiment of the invention, when the proliferation inhibition efficiency of the anti-cancer agent 1 for each cancer cell subgroup is set by the setting unit 102a, the opposite pattern of proliferation inhibition efficiency of the anti-cancer agent 2 is set by the setting unit 102a, and they are consequently selected as an optimum combination by the optimizing unit 102c to be described below, thus design of having anti-cancer agents that are used simultaneously to evenly inhibit the proliferation of the cancer cells can be achieved. Such calculation is usable for selection of pharmaceuticals in clinical practice and it allows a pharmaceutical company to perform the calculation of an anti-cancer agent that is commercially available at present or under development with a screening condition that they are recommended as an anti-cancer agent to be administered as a combination.

Further, with regard to the cell lines from which measured data stored in the measurement database 106a of the embodiment of the invention is collected, standard cancer cell lines exhibiting the most similar drug response to that of an identified cancer cell subgroup, in which the response is determined in certain form by examining the diversity of cancer cells in a patient, may be selected as substitute cells (that is, surrogate cells) for screening. For example, the cell lines from which measured data stored in the measurement database 106a of the embodiment of the invention is collected may be selected as surrogate cell lines exhibiting the most similar response to known cancer cell lines, as illustrated in FIG. 15, based on the result of screening (for example, screening based on pharmaceuticals) each small section of the samples obtained from a surgically removed tumor or by multi-point sampling using needle or biopsy, and performing gene analysis (for example, sequencing and expression profile analysis).

Back to FIG. 8, the setting unit 102a calculates the standard deviation indicating the extent of variation in the yield which has been set in the step SC-1 for each cells and set the standard deviation as variables (risk)(step SC-2). Herein, the setting unit 102a may calculate calculation of standard deviation in expected yield and yield variation as probability density for each culture condition.

The setting unit 102a projects (maps) the yield set in the step SC-1 and the risk set in the step SC-2 into a yield risk space as coordinates of the cells or anti-cancer agent (step SC-3).

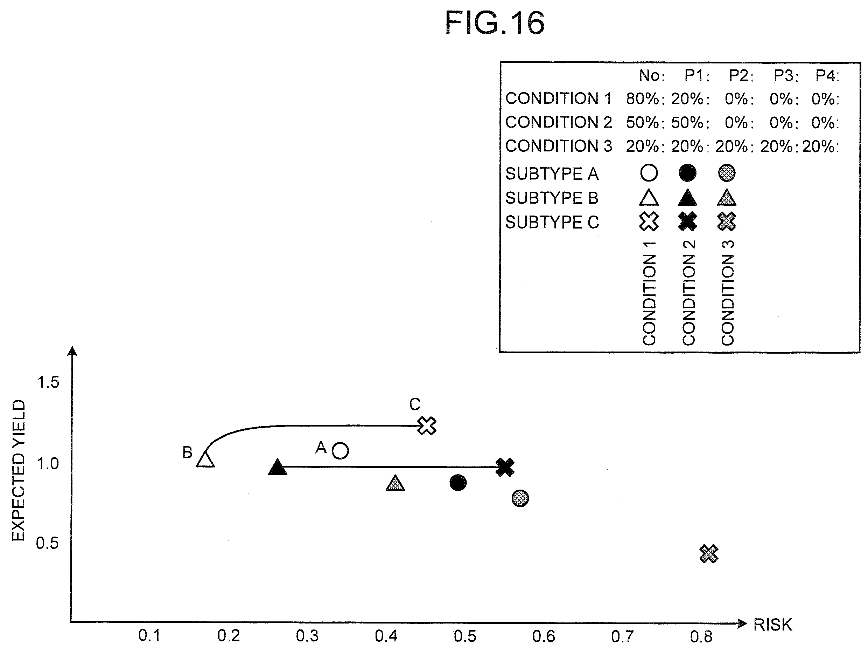

Herein, referring to FIG. 16, one example of projection into the yield risk space according to the embodiment of the invention will be described. FIG. 16 is a diagram for illustrating an example of projection into the yield risk space according to the embodiment of the invention.

As illustrated in FIG. 16, the setting unit 102a projects, based on increase ratio for each cells of the subtypes A, B, and C without disturbance or with disturbance (P) 1 to 4 described in FIG. 9 to FIG. 11 under the culture conditions 1 to 3 and probability of occurrence without disturbance or with disturbance (P) 1 to 4 under the culture conditions 1 to 3, yield and risk of the cells of subtypes A, B, and C under the culture conditions 1 to 3 into the yield risk space.

Back to FIG. 8, the variable range calculating unit 102b calculates a portfolio selectable range, which is a variable range of combination (portfolio) of multiple cells or anti-cancer agents, based on coordinates in a yield risk space projected by the setting unit 102a (step SC-4).

Further, the optimizing unit 102c calculates optimum combination (portfolio) in the variable ranges using an optimization method based on a pre-determined indifference curve, specifies most effective combination of cancer cells cultured under which condition (that is, administered with an anti-cancer agent), and the like, and selects an optimum combination of cancer agent from multiple cancer agents (step SC-5). The process is terminated thereafter. Accordingly, the user such as a pharmaceutical company can decide which candidate compound should be further followed as a subject of development.

### Other Embodiments

The embodiment of the present invention is explained above. However, the present invention may be implemented in various different embodiments other than the embodiment described above within a technical scope described in claims.

For example, an example in which the cell selecting apparatus 100 performs the processing as a standalone apparatus is explained. However, the cell selecting apparatus 100 can be configured to perform processes in response to request from the client terminal (which is a separate case from the cell selecting apparatus 100) and return the process results to the client terminal.

All the automatic processes explained in the present embodiment can be, entirely or partially, carried out manually. Similarly, all the manual processes explained in the present embodiment can be, entirely or partially, carried out automatically by a known method.

The process procedures, the control procedures, specific names, information including registration data for each process and various parameters such as search conditions, display example, and database construction, mentioned in the description and drawings can be changed as required unless otherwise specified.

Further, each constitutional element of the cell selecting apparatus 100 is based on functional concept, and it is not necessarily constituted physically as illustrated in the figures.

For example, the process functions performed by each device of the cell selecting apparatus 100, especially the each process function performed by the control unit 102, can be entirely or partially realized by CPU and a computer program executed by the CPU or by a hardware using wired logic. The computer program, recorded on a non-transitory computer readable recording medium including programmed commands for causing a computer to execute the method of the present invention, can be mechanically read by the cell selecting apparatus 100 as the situation demands. In other words, the storage unit 106 such as read-only memory (ROM) or hard disk drive (HDD) stores the computer program that can work in coordination with an operating system (OS) to issue commands to the CPU and cause the CPU to perform various processes. The computer program is first loaded to the random access memory (RAM), and forms the control unit in collaboration with the CPU.

Further, the computer program may be stored in an application program server which is connected to the cell selecting apparatus 100 via any network, and if necessary, part or entire program can be downloaded.

The computer program may be stored in a computer-readable recording medium, or may be structured as a program product. Here, the "recording medium" includes any "portable physical medium" such as a memory card, a USB (Universal Serial Bus) memory, an SD (Secure Digital) card, a flexible disk, an optical disk, a ROM, an EPROM (Erasable Programmable Read Only Memory), an EEPROM (Electronically Erasable and Programmable Read Only Memory), a CD-ROM (Compact Disk Read Only Memory), an MO (Magneto-Optical disk), a DVD (Digital Versatile Disk), and a Blu-ray Disc.

"Program" refers to a data processing method written in any computer language and written method, and can have software codes and binary codes in any format. The "program" can be a dispersed form in the form of a plurality of modules or libraries, or can perform various functions in collaboration with a different program such as the OS. Any known configuration in the each device according to the embodiment can be used for reading the recording medium. Similarly, any known process procedure for reading or installing the computer program can be used.

Various databases (the measurement database 106a) stored in the storage unit 106 is a storage unit such as a memory device such as a RAM or a ROM, a fixed disk device such as a HDD, a flexible disk, and an optical disk, and stores therein various programs, tables, databases, and web page files used for providing various processing or web sites.

The cell selecting apparatus 100 may be structured as an information processing apparatus such as known personal computers or workstations, or may be structured by connecting any peripheral devices to the information processing apparatus. Furthermore, the cell selecting apparatus 100 may be realized by mounting software (including programs, data, or the like) for causing the information processing apparatus to implement the method according of the invention.

The distribution and integration of the device are not limited to those illustrated in the figures. The device as a whole or in parts can be functionally or physically distributed or integrated in an arbitrary unit according to various attachments or how the device is to be used. That is, any embodiments described above can be combined when implemented, or the embodiments can selectively be implemented.

### INDUSTRIAL APPLICABILITY

As described in detail in the above, the invention is capable of providing the cell selecting apparatus, the cell selecting method, and the computer program product for selecting an optimum combination of cells or microorganisms by mapping, in a yield risk space, a portfolio including production outputs based on cell proliferation rate, production of biomass by microorganisms, or the like, and variables indicating the extent of the variation of production outputs, that are calculated from suppressed proliferation, reduced production outputs, or the like considering multiple disturbances and probability of its occurrence.

### EXPLANATIONS OF LETTERS OR NUMERALS

- 100: Cell selecting apparatus

- 102: Control unit
- 102a: Setting unit
- 102b: Variable range calculating unit
- 102c: Optimizing unit
- 106: Storage unit
- 106a: Measurement database

## Claims

1. An apparatus (100) configured to determine an optimum combination of multiple cells or cell culturing conditions, comprising a storage unit (106) and a control unit (102), wherein
the storage unit (106) stores data about cell proliferation or biomass production for each cell and each condition, and the control unit (102) includes:
a setting unit (102a) configured to set, for each combination of multiple cells or cell culturing conditions a production output based upon the stored data and a variable indicating an extent of variation of the production output;
a variable range calculating unit (102b) configured to calculate a variable range of the production output and the variable for different combinations of multiple cells or conditions based on the production output and the variable set by the setting unit (102a); and
an optimizing unit (102c) configured to calculate an optimum combination of multiple cells or cell culturing conditions within the variable range by calculating a combination representing maximum production as a function of the variable within the variable range by using an optimization method.

2. The apparatus (100) according to claim 1, wherein the variable is standard deviation of the production output.

3. The apparatus (100) according to claim 1 or 2, wherein the variable range calculating unit (102b) is arranged to calculate, based on the production output and the variable, correlations and anti-correlations among cells by a method for calculating covariance, and to calculate a variable range of the production output and the variable in case of combining the multiple cells or conditions.

4. The apparatus (100) according to any one of claims 1 to 3, wherein the optimizing unit (102c) is arranged to calculate a combination closest to effective frontier for maximizing the production output under the constant variable within the variable range by using the optimization method.

5. The apparatus (100) according to any one of claim 1 to 4, wherein the optimization method is Pareto optimization, genetic algorithm, least square method, or a suitable probabilistic or analytical optimization method.

6. The apparatus (100) according to any one of claims 1 to 5, wherein the multiple cells are the cells of multiple types.

7. The apparatus (100) according to any one of claims 1 to 5, wherein the multiple cells are of a single type, and the cell selecting apparatus (100) is configured to determine an optimum combination of cell culturing conditions.

8. The apparatus (100) according to any one of claims 1 to 7, wherein the cells are microorganisms.

9. The apparatus (100) according to any one of claims 1 to 7, wherein the cells are cancer cells.

10. The apparatus (100) according to any one of claims 1 to 9, wherein the measured data is GI50 that is a concentration of a compound for inhibiting cell proliferation by 50% and the production output is a reciprocal number of GI50.

11. A method of determining an optimum combination of multiple cells or cell culturing conditions by an apparatus (100) according to claim 1, the method comprising:
a setting step (SA-1) of setting, for each combination of multiple cells or cell culturing conditions, a production output based upon the stored data and a variable indicating an extent of variation of the production output;
a variable range calculating step (SA-2) of calculating a variable range of the production output and the variable for different combinations of multiple cells or conditions based on the production output and the variable set at the setting step; and
an optimizing step (SA-3) of calculating an optimum combination of multiple cells or cell culturing conditions within the variable range by calculating a combination representing maximum production as a function of the variable within the variable range by using an optimization method.

12. A computer-readable medium storing computer program instructions which, when executed on a computer, cause the computer to perform a method according to claim 11.

## Patentansprüche

1. Vorrichtung (100), die eingerichtet ist zum Bestimmen einer optimalen Kombination von Mehrzellen- oder Zellkultivierungsbedingungen, umfassend eine Speichereinheit (106) und eine Steuereinheit (102), wobei
die Speichereinheit (106) Daten über Zellwucherung oder Biomassenproduktion für jede Zelle und jede Bedingung speichert, und die Steuereinheit (102) enthält:
eine Einstelleinheit (102a), die eingerichtet ist zum Einstellen, für jede Kombination von Mehrzellen- oder Zell-Kultivierungsbedingungen, einer Produktionsausgabe basierend auf den gespeicherten Daten und einer Variable, die ein Ausmaß einer Variation der Produktionsausgabe anzeigt;
eine variable Bereich-Berechnungseinheit (102b), die eingerichtet ist zum Berechnen eines variablen Bereichs der Produktionsausgabe und der Variable für verschiedene Kombinationen von Mehrzellen oder Bedingungen basierend auf der Produktionsausgabe und der durch die Einstelleinheit (102a) eingestellten Variable; und
eine Optimierungseinheit (102c), die eingerichtet ist zum Berechnen einer optimalen Kombination von Mehrzellen- oder Zell-Kultivierungsbedingungen innerhalb des variablen Bereichs durch Berechnen einer Kombination, die eine maximale Produktion darstellt, als eine Funktion der Variable innerhalb des variablen Bereichs durch Verwenden eines Optimierungsverfahrens.

2. Vorrichtung (100) nach Anspruch 1, wobei die variable eine Standardabweichung der Produktionsausgabe ist.

3. Vorrichtung nach Anspruch 1 oder 2, wobei die variable Bereich-Berechnungseinheit (102b) ausgebildet ist zum Berechnen, basierend auf der Produktionsausgabe und der variablen, von Korrelationen und Anti-Korrelationen unter Zellen durch ein Verfahren zum Berechnen einer Kovarianz, und zum Berechnen eines variablen Bereichs der Produktionsausgabe und der Variable im Fall eines Kombinierens der Mehrzellen und Bedingungen.

4. Vorrichtung (100) nach einem der Ansprüche 1 bis 3, wobei die Optimierungseinheit (102c) ausgebildet ist zum Berechnen einer Kombination am nächsten zu einer effektiven Grenze zum Maximieren der Produktionsausgabe unter der konstanten Variable innerhalb des variablen Bereichs durch Verwenden des Optimierungsverfahrens.

5. Vorrichtung (100) nach einem der Ansprüche 1 bis 4, wobei das Optimierungsverfahren eine Pareto-Optimierung, ein genetischer Algorithmus, ein Verfahren kleinster Quadrate oder ein geeignetes wahrscheinlichkeitstheoretisches oder analytisches Optimierungsverfahren ist.

6. Vorrichtung (100) nach einem der Ansprüche 1 bis 5, wobei die Mehrzellen die Zellen mehrerer Typen sind.

7. Vorrichtung (100) nach einem der Ansprüche 1 bis 5, wobei die Mehrzellen ein einzelner Typ sind und die Zellauswahlvorrichtung (100) eingerichtet ist zum Bestimmen einer optimalen Kombination von Zellkultivierungsbedingungen.

8. Vorrichtung (100) nach einem der Ansprüche 1 bis 7, wobei die Zellen Mikroorganismen sind.

9. Vorrichtung (100) nach einem der Ansprüche 1 bis 7, wobei die Zellen Krebszellen sind.

10. Vorrichtung (100) nach einem der Ansprüche 1 bis 9, wobei die gemessenen Daten GI50 sind, was eine Konzentration einer Komponente zum Verhindern einer Zellwucherung um 50% ist, und die Produktionsausgabe eine reziproke Zahl von GI50 ist.

11. Verfahren zum Bestimmen einer optimalen Kombination von Mehrzellen- oder Zell-Kultivierungsbedingungen durch eine Vorrichtung (100) nach Anspruch 1, wobei das Verfahren umfasst:
einen Einstellschritt (SA-1) zum Einstellen, für jede Kombination von Mehrzellen- oder Zell-Kultivierungsbedingungen, einer Produktionsausgabe basierend auf den gespeicherten Daten und einer Variable, die ein Ausmaß einer Variation der Produktionsausgabe anzeigt;
einen variable Bereich-Berechnungsschritt (SA-2) zum Berechnen eines variablen Bereichs der Produktionsausgabe und der Variable für verschiedene Kombinationen von Mehrzellen oder Bedingungen basierend auf der Produktionsausgabe und der durch den Einstellschritt eingestellten Variable; und
einen Optimierungsschritt (SA-3) zum Berechnen einer optimalen Kombination von Mehrzellen- oder Zell-Kultivierungsbedingungen innerhalb des variablen Bereichs durch Berechnen einer Kombination, die eine maximale Produktion darstellt, als eine Funktion der Variable innerhalb des variablen Bereichs durch Verwenden eines Optimierungsverfahrens.

12. Computerlesbares Medium, das Computerprogrammbefehle speichert, die, wenn von einem Computer ausgeführt, den Computer veranlassen, ein Verfahren nach Anspruch 11 auszuführen.

## Revendications

1. Appareil (100) configuré pour déterminer une combinaison optimale de multiples cellules ou conditions de culture de cellules, comprenant une unité de mémoire (106) et une unité de commande (102), dans lequel :
l'unité de mémoire (106) met en mémoire des données sur la prolifération de cellules ou la production de biomasse pour chaque cellule et chaque condition, et l'unité de commande (102) comprend :
une unité de réglage (102a) configurée pour régler, pour chaque combinaison de multiples cellules ou conditions de culture de cellules, un rendement de production sur la base des données mises en mémoire et une variable indiquant le degré de variation du rendement de production ;
une unité de calcul de plage de variables (102b) configurée pour calculer une plage de variables du rendement de production et la variable pour différentes combinaisons de multiples cellules ou conditions sur la base du rendement de production et de la variable réglés par l'unité de réglage (102a) ; et
une unité d'optimisation (102c) configurée pour calculer une combinaison optimale des multiples cellules ou conditions de culture de cellules dans la plage de variables en calculant une combinaison représentant une production maximale en fonction de la variable dans la plage de variables en faisant appel à un procédé d'optimisation.

2. Appareil (100) selon la revendication 1, dans lequel la variable est un écart-type du rendement de production.

3. Appareil (100) selon la revendication 1 ou 2, dans lequel l'unité de calcul de plage de variables (102b) est configurée pour calculer, sur la base du rendement de production et de la variable, des corrélations et des anti-corrélations parmi les cellules par un procédé de calcul de covariance, et pour calculer une plage de variables du rendement de production et la variable dans le cas d'une combinaison des multiples cellules ou conditions.

4. Appareil (100) selon l'une quelconque des revendications 1 à 3, dans lequel l'unité d'optimisation (102c) est conçue pour calculer la combinaison la plus proche d'une frontière efficace pour augmenter au maximum le rendement de production dans le cadre de la variable constante dans la plage de variables en faisant appel au procédé d'optimisation.

5. Appareil (100) selon l'une quelconque des revendications 1 à 4, dans lequel le procédé d'optimisation est une optimisation de Pareto, un algorithme génétique, la méthode des moindres carrés ou un procédé d'optimisation probabiliste ou analytique approprié.

6. Appareil (100) selon l'une quelconque des revendications 1 à 5, dans lequel les multiples cellules sont des cellules de multiples types.

7. Appareil (100) selon l'une quelconque des revendications 1 à 5, dans lequel les multiples cellules sont des cellules d'un seul type et l'appareil de sélection de cellules (100) est configuré pour déterminer une combinaison optimale de conditions de culture de cellules.

8. Appareil (100) selon l'une quelconque des revendications 1 à 7, dans lequel les cellules sont des microorganismes.

9. Appareil (100) selon l'une quelconque des revendications 1 à 7, dans lequel les cellules sont des cellules cancéreuses.

10. Appareil (100) selon l'une quelconque des revendications 1 à 9, dans lequel les données mesurées sont le GI50 qui correspond à une concentration d'un composé permettant d'inhiber la prolifération cellulaire de 50 % et le rendement de production est un nombre inverse du GI50.

11. Procédé de détermination d'une combinaison optimale de multiples cellules ou conditions de culture de cellules par un appareil (100) selon la revendication 1, le procédé comprenant :
une étape de réglage (SA-1) pour régler, pour chaque combinaison de multiples cellules ou conditions de culture de cellules, un rendement de production sur la base des données mises en mémoire et d'une variable indiquant un degré de variation du rendement de production ;
une étape de calcul de plage de variables (SA-2) pour calculer une plage de variables du rendement de production et la variable pour différentes combinaisons de multiples cellules ou conditions sur la base du rendement de production et de la variable réglés au cours de l'étape de réglage ; et
une étape d'optimisation (SA-3) pour calculer une combinaison optimale de multiples cellules ou conditions de culture de cellules dans la plage de variables en calculant une combinaison représentant une production maximale en fonction de la variable dans la plage de variables en faisant appel à un procédé d'optimisation.

12. Support lisible par ordinateur mettant en mémoire des instructions de programmation informatiques qui, une fois exécutées sur un ordinateur, amène l'ordinateur à effectuer un procédé selon la revendication 11.
